# EUROPÄISCHE PATENTSCHRIFT

(11) **EP 0 962 455 B1**
(45) Veröffentlichungstag und Bekanntmachung des Hinweises auf die Patenterteilung: **06.11.2002**
(21) Anmeldenummer: 99109890.6
(22) Anmeldetag: 20.05.1999
(51) Int. Cl.: C07D 273/04, C07D 251/34, C08G 18/02

(54) **Verfahren zur Herstellung Iminooxadiazindiongruppen enthaltender Polyisocyanate**
Process for the preparation of imino-oxadiazine-dione groups containing polyisocyanates
Procédé pour la préparation de polyisocyanates contenant des groupes imino-oxadiazine-dione

(30) Priorität: 02.06.1998 DE 19824485; 02.06.1998 DE 19824490
(43) Veröffentlichungstag der Anmeldung: 08.12.1999
(73) Patentinhaber: Bayer Aktiengesellschaft, 51368 Leverkusen (DE)
(72) Erfinder: Richter, Frank, Dr., 51373 Leverkusen (DE); Stelter, Eberhard, Dr., 50735 Köln (DE); Litz, Wilfried, Dr., 51107 Köln (DE); Groth, Stefan, Dr., 51373 Leverkusen (DE)

(56) Entgegenhaltungen:
- EP-A- 0 355 479
- EP-A- 0 379 914
- EP-A- 0 798 299
- EP-A- 0 896 009

## Beschreibung

Iminooxadiazindiongruppen (asymmetrische Trimere) enthaltende Polyisocyanate sind interessante, hochwertige Rohstoffe u.a. für die Herstellung von Polyurethan-Lacken und Beschichtungen (z.B. DE-A 19 611 849). Sie sind als Nebenkomponente ein ständiger Begleiter der lange bekannten Isocyanurate (symmetrischen Trimeren).

Isocyanat-Oligomere mit signifikant erhöhtem Iminooxadiazindiongehalt sind Gegenstand einer eigenen älteren Anmeldung (DE-A 19 611 849). Deren vorteilhafte Eigenschaften, z.B. als Rohstoff zur Herstellung von Polyurethan-Lacken und Beschichtungen werden beschrieben. Iminooxadiazindiongruppen enthaltende Polyisocyanate haben für Isocyanat-Oligomere mit mindestens drei NCO-Gruppen eine sehr niedrige Viskosität.

Bei der Übertragung der Herstellung Iminooxadiazindiongruppen enthaltender Isocyanat-Trimerisate mit den in den Beispielen der DE-A 19 611 849 aufgeführten Ammoniumpolyfluorid Katalysatoren vom Labor in den technischen Maßstab hat sich gezeigt, daß der Anteil an asymmetrischem Trimerisat im Trimerengemisch [der Begriff 'Trimerengemisch' steht hier immer für die Summe aus symmetrischen (Isocyanurate) und asymmetrischen Trimeren (Iminooxadiazindione)] schwankt. Die so herstellbaren Produkte können mitunter auch ein hohes Trübungsniveau aufweisen (Trübungswerte, gemessen mit einem Gerät der Fa. Hach, über 1,5 TE(F)).

Thermokinetische Untersuchungen der Trimerisierungsreaktion an Hexamethylendiisocyanat (HDI) mit Ammoniumpolyfluorid-Katalysatoren in einem Reaktionskalorimeter (zu Meßanordung und -prinzip vgl. *J*. *Thermal Anal*. **1983**, *27,* 215-228) ergaben, daß der zeitliche Verlauf der Wärmeentwicklung bei manchen Versuchen vom üblichen Bild, d.i.: verstärkte Wärmeproduktion nach Katalysatorzugabe und anschließender, mehr oder weniger langsamer aber stetiger Abfall der Wärmetönung infolge Katalysatordesaktivierung im Reaktionsgemisch (thermische Zersetzung, Reaktion mit Spurenverunreinigungen im zu oligomerisierenden (Di)isocyanat-(gemisch) etc.) stark abweicht.

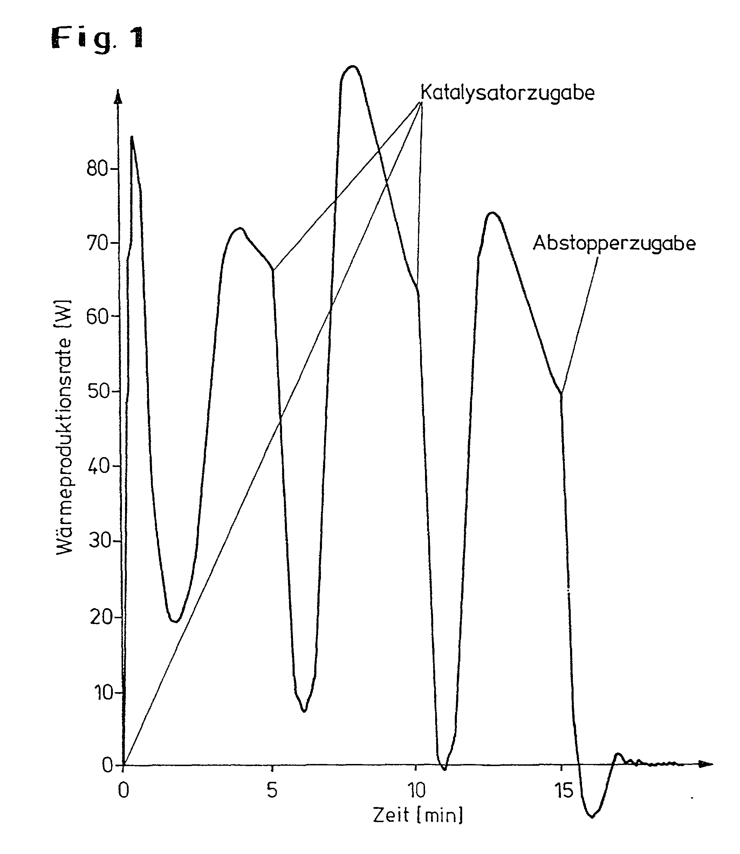

Im Gegensatz dazu tritt häufig zunächst die erwartete schnelle Freisetzung von Reaktionswärme auf, gefolgt von einem eher untypisch schnellen Abklingen und anschließendem erneuten Anspringen der Reaktion. Weitere Katalysatorzugabe beschleunigt überraschenderweise nicht unmittelbar die Reaktion, sondern für einen kurzen Zeitraum unmittelbar nach Zugabe des Katalysators tritt eine Verlangsamung ein, um nach Durchlaufen eines Minimums der Wärmeproduktionsrate ohne ersichtlichen äußeren Anlaß erneut wieder 'durchzustarten' (Beispiel 2 und Abb. 1).

Dieses Phänomen wird allerdings durchaus nicht immer beobachtet. Es ist auch nicht von der Reaktionstemperatur abhängig. Wird kein anomaler zeitlicher Verlauf der Wärmeproduktionskurve beobachtet, ist der Anteil an asymmetrischen Trimeren auf dem aus Laborversuchen gewohnten, hohen Niveau (d.h. über 30 mol-% im Trimerengemisch). Beobachtet man den o.g. anomalen Verlauf der Wärmeproduktionskurve, resultieren Produkte mit weit niedrigerem Iminooxadiazindiongehalt.

Offenbar bildet/bilden sich in kaum vorhersehbarer Weise erst während der Reaktion aus dem zugegebenen Ammoniumpolyfluorid durch den Einfluß des zu oligomerisierenden Isocyanates bzw. von Nebenprodukten in letzterem die eigentlich katalytisch aktive/n Spezies, die je nach Reaktionstyp (normal versus anomal im Sinne der eben gegebenen Beschreibung) unterschiedliche Produkt(gemische)e liefert/liefern.

Dieser Umstand erschwert eine gezielte reproduzierbare technische Herstellung hochwertiger Lackpolyisocyanate mit reproduzierbaren Eigenschaften wie Viskosität, NCO-Gehalt, Farbzahl, Trübung etc. außerordentlich.

Aufgabe der Erfindung war es daher, ein reproduzierbares Verfahren zur Verfügung zu stellen, das nicht mit den o.g. Unwägbarkeiten behaftet ist. Die Umsetzung sollte vorhersehbar in unmittelbarer Abhängigkeit von der eingesetzten Katalysatormenge durchführbar sein, und die Wärmeproduktion der exothermen Reaktion sollte gleichmäßig auftreten und gleichmäßig abführbar sein. Die Verfahrensprodukte sollten in erwarteter, einheitlicher Zusammensetzung und Qualität hestellbar sein.

Diese Aufgabe konnte durch das erfindungsgemäße Verfahren, die Katalyse der Isocyanat-Trimerisierung durch quaternäre Phosphoniumpolyfluoride, gelöst werden.

Gegenstand der vorliegenden Erfindung ist ein Verfahren zur Herstellung von Trimerisat-Polyisocyanaten die mindestens 30 mol-% an Iminooxadiazindiongruppen (asymmetrische Trimere) im Trimerengemisch enthalten durch katalytisch induzierte Trimerisierung organischer Di- oder Polyisocyanate mit einem (mittleren) Molekulargewicht von 140 - 600 g/mol mit unabhängig voneinander aliphatisch, cycloaliphatisch und/oder araliphatisch gebundenen Isocyanatgruppen, dadurch gekennzeichnet, daß als Trimerisierungskatalysator quaternäre Phosphoniumpolyfluoride der Formel R₄P⁺ F^{-·} n(HF), wobei R für gleiche oder verschiedene, gegebenenfalls verzweigte, aliphatische, aromatische und/oder araliphatische C₁-C₂₀ Reste steht und gegebenenfalls zwei oder mehrere Substituenten R untereinander und mit dem Phosphoratom auch gesättigte oder ungesättigte Cyclen bilden können und n beliebige Werte zwischen 0,1 und 20 annehmen kann, in Abmischung mit Alkohol (gemische)en eines (mittleren) Molekulargewichbbereiches von 32-250 g/mol eingesetzt werden und die Konzentration des quaternären Phosphoniumpolyfluorides 10 Masse-% in der Mischung nicht unterschreitet. (Der Terminus 'Trimerengemisch' bezieht sich immer auf die Mischung aus Isocyanurat und Iminooxadiazindion-Strukturen).

Bevorzugt ist das Verfahren, bei dem als zu trimerisierende Isocyanatkomponente aliphatische Diisocyanate eines Molekulargewicht(sbereich)es von 140 - 300 g/mol als reine Verbindungen oder in beliebiger Abmischung untereinander eingesetzt werden wobei die Verfahrensprodukte mindestens 35 %, bevorzugt mindestens 40 mol-% an Iminooxadiazindiongruppen (asymmetrische Trimere) im Trimerengemisch enthalten.

Zur Durchführung des erfindungegemäßen Verfahrens kann man reine Verbindungen bzw. beliebige Mischungen von Verbindungen der Summenformel R₄P⁺ F^{-. n(HF)} einsetzen, wobei R für gleiche oder verschiedene, gegebenenfalls verzweigte, gegebenenfalls substituierte, aliphatische, aromatische und/oder araliphatische C₁-C₂₀ Reste steht. Kommerziell erhältlich, zumindest in Form ihrer Salze mit anderen als Polyfluorid-Gegenionen, die sich aber leicht in die Polyfluoridform überführen lassen, z.B. Chloride, Bromide, Iodide, (Hydrogen)sulfate; vgl. u.a. *Synthesis* **1988**, *12*, 953-5 und Beispiel 1, sind beispielsweise Tetrakis(hydroxymethyl)phosphoniumchlorid und -sulfat, Tetraethyl-, Tetrabutyl-, Tetraoctyl-, Tetrakis(hexadecyl)-, Tributyl(tetradecyl)-, Tributyl(hexadecyl)- und Trioctyl(octadecyl)phosphoniumchlorid bzw. -bromid und/oder -iodid.

Da diese Verbindungen in der Regel als reine Stoffe fest sind (vgl. Beispiel 1), benötigt man für ihren Einsatz bei der erfindungsgemäßen Isocyanat-Trimerisierung in der Regel Katalysatorlösungsmittel. Hierfür eignen sich beispielsweise geradkettige und verzweigte, primäre, sekundäre und tertiäre Alkohole mit ein bis zwanzig C-Atomen, bevorzugt ein bis acht C-Atomen, z.B. Methanol, Ethanol, n-sowie iso-Propanol, 1- und 2-Butanol, iso-Butanol und 2-Ethylhexanol.

Auch die Verwendung von Triphenyl(alkyl)derivaten ist prinzipiell möglich, wenngleich sie aufgrund ihrer im Vergleich zu den rein aliphatisch substituierten Vertretern schlechteren Löslichkeit in den für den Einsatz in der Isocyanatoligomerisierung geeigneten Lösungsmitteln, insbesondere Alkohole, weniger bevorzugt sind (Beispiele lc und 3-15).

Obwohl ganz allgemein der Einsatz von Polyfluoriden aus der DE-A 19 611 849 bekannt ist, läßt sich für den Fachmann hieraus kein Schluß auf den besonders vorteilhaften Einsatz von quaternären Phosphoniumpolyfluoriden für die gut reproduzierbare und vor allem unter allen Herstellbedingungen trübungsfreie Herstellung von Polyisocyanaten mit einem besonders hohen Iminooxadiazindiongruppengehalt ziehen.

Sämtliche Ausführungsbeispiele der DE-A 19 611 849 beziehen sich auf die Katalyse mit Polyfluoriden auf Basis quaternärer Ammoniumsalze die mit den weiter oben aufgeführten Nachteilen behaftet ist. Die besondere Rolle, die der Art des Kations im Katalysatormolekül zukommt, wird nicht diskutiert.

Die Beobachtung, daß die Art des Gegenions für das Polyfluoridanion (hier: quaternäres Phosphonium) einen entscheidenden Einfluß auf den reproduzierbaren Verlauf der gewünschten Reaktion unter Bildung hochwertiger Produkte mit hohem Iminooxadiazindiongruppengehalt und einheitlicher Qualität (z.B. geringe Trübung) hat, ist außerordentlich überraschend und geht aus der DE-A 19 611 849 in keiner Weise hervor.

Ferner wird gelegentlich der Einsatz von, gegebenenfalls immobilisierten, gegebenenfalls in situ hergestellten (Phasentransferkatalyse, vgl. *Isr*. *J*. *Chem.* **1985**, 26, 222-224, Phosphoniumfluoride werden dort allerdings nicht beschrieben), Phosphoniumfluoriden zur Isocyanat-Trimerisierung vorgeschlagen: DE-A 39 02 078, DE-A 38 27 596, EP-A 0 315 692.

In der EP-A 0 315 692 werden, dem Konzept der Phasentransferkatalyse folgend, Kaliumfluorid-katalysierte Prozesse zur Herstellung von Substanzen mit Isocyanurat-Ringstrukturen beschrieben, wobei u.a. die simultane Anwesenheit von Phosphonium-Verbindungen zur 'Steigerung der Effizienz der Reaktion' vorgeschlagen wird. Polyfluoride werden nicht erwähnt. Auch in den Ausführungsbeispielen dieser Patentschrift finden sich keinerlei Hinweise auf den Einsatz von Phosphoniumsalzen. Außerdem wird hauptsächlich auf die Trimerisierung aromatischer Isocyanate (TDI, MDI) eingegangen und nur anhand der Reaktion von n-Butylisocyanat mit KF an zwei Beispielen die katalytische Wirkung des Kaliumfluorides an sich (Bsp. 1 der EP-A 0 315 692) bzw. in Gegenwart von quaternärem Ammoniumsalz (Benzyltrimethylammoniumchlorid, Bsp. 5 der EP-A 0 315 692) zur Trimerisierung von Isocyanaten mit aliphatisch gebundenen NCO-Gruppen unter Isocyanuratbildung demonstriert. Die hohe Reaktionstemperatur (120°C) sowie die vergleichsweise langen Reaktionszeiten (8 h im Bsp. 1, 4 h im Bsp. 5 der EP-A 0 315 692) bei hoher Katalysatorkonzentration, die technisch unvorteilhafte Abtrennung der festen Kaliumsalzkomponenten nach der Reaktion durch Filtration (Bsp. 1 der EP-A 0 315 692) bzw. Auswaschen mit Wasser (Bsp. 5 der EP-A 0 315 692), letzteres wäre ohnehin prohibitiv für die Herstellung von Produkten, die freie Isocyanatgruppen enthalten, sowie der Umstand, daß durch die (mit)beanspruchte Verwendung von Phosphoniumsalz neben Kaliumfluorid eine ständige 'Extraktion' von Fluoridionen aus der unlöslichen, anorganischen Phase, die als eigentlicher Katalysator bezeichnet wird, in die organische, Isocyanat enthaltende Phase erfolgt, lassen die Methode generell als wenig vorteilhaft und technisch kaum realisierbar erscheinen.

Ferner wird in der EP-A 235 388 ein Verfahren zur Herstellung gemischter Isocyanat-Polycarboxylsäure/Polycarboxylsäureanhydrid-Folgeprodukte unter Katalyse mit Alkalimetallfluoriden bei gleichzeitiger Gegenwart von quaternären Oniumsalzen beschrieben. Auf Seite 2, Spalte 2, Zeilen 21-23 der EP-A 235 388 wird allerdings explizit darauf verwiesen, daß keine Produkte aus der Reaktion der NCO-Gruppen untereinander entstehen. Genau diese Produkte (asymmetrische und symmetrische Trimerisate) sind Gegenstand der vorliegenden Anmeldung.

Es findet sich, mit Ausnahme der DE-A 19 611 849, in keiner Vorveröffentlichung ein Verweis auf den besonders vorteilhaften Einsatz von Polyfluoriden, d.h. HF-Fluorid-Addukten, zur Isocyanat-Modifizierung.

Hinzu kommt, daß beispielsweise in der DE-A 39 02 078 Phosphoniumfluoride an mehreren Stellen ausdrücklich gegenüber Ammoniumspezies als 'weniger bevorzugt' herausgestellt werden (Seite 3, Zeilen 32/33, Zeilen 60/61, Seite 4, Zeile 12) und weiterhin darauf verwiesen wird, daß in den resultierenden Produkten 'der Iminooxadiazindionanteil von untergeordneter Größe' bleibt (S. 4, Zeilen 51-52). Die Ausführungsbeispiele 6 bis 9 der DE-A 39 02 078, in denen über die anteilige Bildung von Iminooxadiazindionen neben Isocyanurat und Oxadiazintrion, als den beiden Hauptprodukten der Reaktion, berichtet wird, legen zudem eher den Schluß nahe, daß einerseits die Iminooxadiazindionbildung der Gegenwart von CO₂ bei der Trimerisierungsreaktion bedarf und andererseits eine eher unerwünschte Nebenreaktion ist.

Es war deshalb für den Fachmann auf der Basis des vorbeschriebenen Standes der Technik nicht ersichtlich, daß im organischen Medium vollständig lösliche, quaternäre Phosphoniumpolyfluoride besonders vorteilhaft zur gut reproduzierbaren Herstellung trübungsfreier Isocyanattrimerisatharze mit hohem Iminooxadiazindiongruppenanteil im Trimerengemisch geeignet sein könnten.

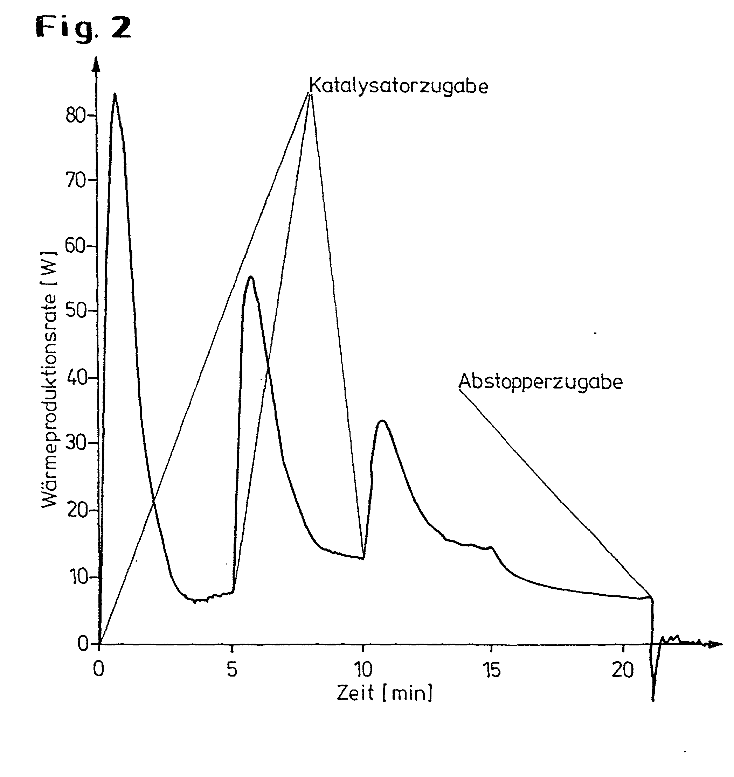

Besonders überraschend ist die Beobachtung, daß, in Abweichung zur Katalyse mit den chemisch sehr ähnlichen quaternären Ammoniumpolyfluoriden, beim erfindungsgemäßen Einsatz von quaternären Phosphonium(poly)fluoriden zur Isocyanattrimerisierung in thermokinetischen Messungen immer der 'normale' Reaktionsverlauf, d.h. das erwartete Bild von verstärkter Wärmeproduktion nach Katalysatorzugabe und anschließender, mehr oder weniger langsamer aber stetiger Abfall infolge Katalysatordesaktivierung im Reaktionsgemisch, z.B. durch Reaktion des Katalysators mit Spurenverunreinigungen im zu oligomerisierenden (Poly)isocyanat(gemisch), zu beobachten ist (Beispiel 3-1 und Abbildung 2).

Diese Effekte sind keineswegs auf die literaturbekannte höhere thermische Stabilität von Tetraorganyl-phosphonium- im Vergleich zu den ähnlichen -ammoniumsalzen zurückzuführen (vgl. z.B. Methoden der Organischen Chemie, 'Houben-Weyl', 4. Aufl., G. Thieme Verlag, Stuttgart, Bd. XII/1, S. 47 bzw. ebenda, Bd. XI/2, S. 633 ff), wie Messungen zur Isocyanat-Trimerisierung bei verschiedenen Temperaturen belegen. Ohnehin erfolgt die Trimerisierungsreaktion vorzugsweise bei Temperaturen, die weder bei den Ammonium- noch bei den Phosphoniumpolyfluoriden Anzeichen einer Zersetzung in differenzthermoanalytischen Messungen (DTA) lieferten.

Offenbar steht die Bildung der eigentlich katalytisch aktiven Spezies ('aktivierter Komplex') aus ursprünglichem Katalysatormolekül und Isocyanatgruppe(n) in Gegenwart eines Überschusses an zu trimerisierendem (Di)isocyanat mit anderen, sehr schwer vorhersagbaren Parametern in Zusammenhang, denen durch die erfindungsgemäße Katalyse mit Phosphonium- anstatt z.B. mit Ammoniumverbindungen erheblich besser und vor allem reproduzierbar entsprochen werden kann.

Der Wert von n in Formel (I) ist nicht kritisch; zwar wird man aus praktischen Erwägungen und auch wegen der unangenehmen physiologischen Eigenschaften des Fluorwasserstoffes diesen nicht in beliebigem molaren Überschuß, bezogen auf vorhandenes Fluorid, F⁻, einsetzen, prinzipiell ist dies aber nicht prohibitiv zur Herstellung von Polyisocyanaten mit hohem Iminooxadiazindionanteil. Selbst ein Katalysatorsystem mit 20-fachem molaren Fluorwaserstoffüberschuß, bezogen auf vorhandenes Fluorid (F⁻) liefert qualitativ einwandfreie Produkte mit hohem Iminooxadiazindionanteil (über 50 mol-% in der Trimerenmischung, Beispiele 3-11 bis 3-13). Allerdings genügen stöchiometrische (n =1) oder unterstöchiometrische HF-Mengen (n = z. B. 0,5) bezogen auf die Menge an Fluoridionen, völlig aus, so daß n im allgemeinen im Bereich von 0,1 bis 2,5 liegt.

Das erfindungsgemäße Verfahren wird in einem Temperaturbereich von 20°C (Zimmertemperatur) bis 200°C, bevorzugt zwischen 30°C und 120°C, besonders bevorzugt zwischen 40°C und 100°C unter anteiliger Umsetzung der beteiligten Isocyanatgruppen des/der Ausgangs(poly)isocyanat(es)/mischung durchgeführt. Der Umsetzungsgrad U_{NCO}, berechnet als Quotient aus der Differenz des NCO-Gehaltes des/der Ausgangs(poly)isocyanat(es)/mischung vor der Trimerisierung und des NCO-Gehaltes der Reaktionsmischung nach Abbruch der Reaktion und dem NCO-Gehalt des/der Ausgangs(poly)isocyanat(es)/mischung vor der Trimerisierung, liegt zwischen 5 % und 60 %, bevorzugt zwischen 10 % und 40 %.

Dabei nicht umgesetztes Monomer kann nach Desaktivierung des Katalysatorsystems nach einem beliebigen Verfahren des Standes der Technik, z.B. durch (Dünnschicht)-destillation oder Extraktion, abgetrennt und anschließend wiederverwendet werden.

Zur Desaktivierung des Katalysatorsystems nach Erreichen des gewünschten U_{NCO} eignen sich prinzipiell alle vorbeschriebenen Methoden des Standes der Technik, wie sie beim Abstoppen der Trimerisierungsreaktion unter Isocyanuratbildung angewandt werden. Das sind z.B. die Zugabe unter- oder auch überstöchiometrischer Mengen an starken Säuren oder Säurederivaten (z.B. Benzoylchlorid, saure Ester der phosphorigen Säure und der Phosphorsäure, diese Säuren selbst etc., nicht jedoch HF), adsorptive Bindung des Katalysators und anschließende Abtrennung durch Filtration, thermische Desaktivierung u.s.w..

Die Entfernung von überschüssigem/n Ausgangsisocyanat/en, sofern es sich bei diesem/n um (ein) niedermolekulare(s), 'monomere(s)' (Di)isocyanat(e) handelt, erfolgt vorzugsweise, wenn die erfindungsgemäßen Verfahrensprodukte für Anwendungen auf dem Polyurethanlack- und beschichtungsmittelsektor bestimmt sind. Hierbei profitiert man von der exellenten Farbzahl und -stabilität der Verfahrensprodukte sowie ihrer hohen Rückspaltstabilität in das/die zugrundeliegende(n) monomere(n) (Di)isocyanat(e).

Zur Herstellung der erfindungsgemäßen Trimeren(mischungen) genügen Katalysatorkonzentrationen, bezogen auf eingesetzte(s) (Poly)isocyanat(mischung) und das Fluoridion (rel. Molmasse 19), zwischen 1 ppm und 1 %, bevorzugt zwischen 1 ppm und 0,1 %, besonders bevorzugt zwischen 1 ppm und 0,05 %.

Nach einer besonderen, kontinuierlich arbeitenden Ausführungsform des erfindungsgemäßen Verfahrens wird die Oligomerisierung in einem Rohrreaktor vorgenommen. Hierbei profitiert man von der sehr geringen Neigung der Phosphoniumpolyfluoridkatalysatoren, auch bei Applikation in hochkonzentrierter Lösung bzw. als reiner Wirkstoff, Gelteilchen im Produkt zu bilden.

Das erfindungsgemäße Verfahren kann sowohl lösungsmittelfrei als auch unter Verdünnung des Ausgangs(poly)isocyanates bzw. der Ausgangs(poly)isocyanat(mischung)e(n) durchgeführt werden. Zur Verdünnung eignen sich hierbei alle, gegenüber NCO-Gruppen inerte organische Verbindungen wie Toluol, Xylol(e), höhere Aromaten, Ester, Ether, Ketone, C₁₂ -C₂₀-Alkylsulfonsäureester sowie Gemische derartiger Lösungsmittel.

Zur Durchführung des erfindungsgemäßen Verfahrens können alle Di- oder Polyisocyanate mit einem (mittleren) Molekulargewicht von 140 - 600 g/mol mit unabhängig voneinander aliphatisch, cycloaliphatisch und/oder araliphatisch gebundenen Isocyanatgruppen in reiner Form oder als beliebige Mischungen untereinander verwendet werden. Beispielhaft seien genannt:
Hexamethylendiisocyanat (HDI), 2-Methylpentan-1,5-diisocyanat (MPDI), 1,3-Bis(isocyanatomethyl)cyclohexan (1,3-H₆-XDI), 3(4)-Isocyanatomethyl-1-methylcyclohexylisocyanat (IMCI); Isophorondiisocyanat (IPDI), Bis(isocyanatomethyl)-norbornan (NBDI), 4-Isocyanatomethyl-1,8-octandiisocyanat (Triisocyanatononan, TIN), 1,3-Bis(isocyanatomethyl)benzol, 1,3-Bis(2-isocyanatopropyl-2)benzol und Bis(4(2)-Isocyanatocyclohexyl)methan (H₁₂MDI, Desmodur® W, Produkt der Bayer AG). Hierbei ist es belanglos, nach welchen Verfahren die vorstehend genannten (Poly)isocyanate hergestellt werden, d.h. mit oder ohne Verwendung von Phosgen.

Bevorzugt werden HDI, MPDI, 1,3-H₆XDI, NBDI sowie Mischungen aus HDI und IPDI eingesetzt.

Es kann weiterhin von Vorteil sein, Gemische bestimmter (Poly)isocyanate im erfindungsgemäßen Verfahren einzusetzen, beispielsweise um dem Anforderungsprofil des jeweiligen Produktes bzw. Produktgemisches optimal zu entsprechen. So werden in vielen Anwendungen, beispielsweise bei der Automobil(erst)lackierung, Polyisocyanatgemische auf Basis, gegebenenfalls verzweigter, linearaliphatischer, z.B. HDI, einerseits und cycloaliphatischer Diisocyanate, z.B. IPDI oder H₁₂MDI (Desmodur® W; Handelsprodukt der Bayer AG), andererseits eingesetzt. Diese Gemische werden in der Regel durch nachträgliches Abmischen von Polyisocyanaten auf Basis von Diisocyanaten des einen mit denen des anderen Typs hergestellt. Es kann aber auch von Vorteil sein, sie durch simultane Mischtrimerisation aus dem entsprechenden Gemisch der monomeren Komponenten herzustellen (EP-A 00 47 452). Viele Polyisocyanate auf Basis cycloaliphatischer Diisocyanate des Standes der Technik sind fest. Sie weisen zuweilen eine derart hohe Schmelzviskosität auf, daß mitunter schon die Monomerenabtrennung durch (Dünnschicht)destillation erhebliche Schwierigkeiten bereitet. Deshalb bedarf es zu ihrer Verarbeitung bzw. mitunter auch bereits bei der Dünnschichtdestillation der Verwendung von Lösungsmitteln bzw. von Fließhilfsmitteln. Will man keine allzu großen Einbußen in Umsetzungsgrad (Harzausbeute) und NCO-Funktionalität bei der Herstellung dieser Polyisocyanate hinnehmen, sind Lösungskonzentrationen um 70 % (Fest)harz, z.B. bei Isocyanurat-Polyisocyanaten auf Basis cycloaliphatischer Diisocyanate, bei gut verarbeitbaren dynamischen Viskositäten zwischen ein und zehn Pa·s (gemessen bei 23°C) marktüblicher Stand der Technik.

Trimerisiert man hingegen Gemische linearer aliphatischer Isocyanate, wie HDI, und cycloaliphatischer Diisocyanate, z.B. IPDI, gemäß dem erfindungsgemäßen Verfahren unter (teilweiser) Iminooxadiazindionbildung, erhält man auch bei Zimmertemperatur fließfähige Produkte (Viskosität bei 23°C unter 100 Pa·s) die zudem bei sukzessiver Lösungsmittelzugabe eine drastisch schnellere Viskositätsabnahme aufweisen, als Produkte entsprechender Zusammensetzung (NCO-Funktionalität, Diisocyanatbasis, mittleres Molekulargewicht) des Standes der Technik (Beispiel 4).

Die nach dem erfindungsgemäßen Verfahren erhältlichen Produkte bzw. Produktgemische stellen mithin vielseitig verwendbare Ausgangsmaterialien zur Herstellung von, gegebenenfalls geschäumte(n), Kunststoffe(n) sowie Lacken, Beschichtungsmitteln, Klebstoffen und Zuschlagstoffen dar.

Vor ihrer Verwendung als Isocyanat-Komponente in Polyurethansystemen können die Verfahrensprodukte gegebenenfalls an den Isocyanatgruppen weiter modifiziert werden, beispielsweise durch Einführung von Urethan-, Harnstoff-, Biuret- und/oder Allophanatgruppen oder durch eine teilweise oder vollständige Umsetzung der NCO-Gruppen mit speziellen, wieder abspaltbaren Verbindungen ('Blockierung'). Hierfür eignen sich beispielsweise Phenole, Lactame wie z.B. ε-Caprolactam, Oxime, Diund Triazole, bestimmte Amine wie z.B. Diisopropylamin, CH-acide Verbindungen wie z.B. Malonsäuredialkylester, Acetessigester etc..

Insbesondere zur Herstellung von gegebenenfalls wasserdipergierbaren Ein- und Zweikomponenten-Polyurethanlacken eignen sich die erfindungsgemäß hergestellten Produkte, gegebenenfalls in NCO-blockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil. So sind die erfindungsgemäßen Verfahrensprodukte auf HDI-Basis auch in hoher Verdünnung in Lacklösungsmitteln stabiler gegen das Auftreten von Ausflockungen bzw. Trübungen, als entsprechende, im wesentlichen Isocyanuratgruppen enthaltende Produkte des Standes der Technik. Ihre Resistenz gegenüber der Einwirkung von Luftfeuchtigkeit (z.B. Hautbildung im offenen Gebinde, mattes Erscheinungsbild von Flächen, die bei hoher Luftfeuchtigkeit und Umgebungstemperatur lackiert wurden, sog. 'downglossing') ist ebenfalls gegenüber den (fast) ausschließlich Isocyanuratgruppen enthaltenden Produkten verbessert.

### Beispiele

Alle Prozentangaben sind, soweit nicht anders vermerkt, Gewichtsprozent.

Mol-% Angaben werden NMR-spektroskopisch ermittelt und beziehen sich immer, so nicht anders ausgewiesen, auf die Summe der durch die Modifizierungsreaktion ('Trimerisierung') entstehenden NCO-Folgeprodukte. Die Messungen erfolgen auf dem Gerät DPX 400 der Fa. Bruker an ca. 5 %igen (¹H-NMR) bzw. ca. 50 %igen (¹³C-NMR) Proben in trokkenem CDCl₃ bei einer Frequenz von 400 MHz (¹H-NMR) bzw. 100 MHz (¹³C-NMR). Als Referenz für die ppm-Skale werden geringe Mengen von Tetramethylsilan im Lösungsmittel mit einer ¹H-chem, Verschiebung von 0 ppm (¹H-NMR) bzw. das Lösungsmittel selbst (CDCl₃) mit einer Verschiebung von 77,0 ppm (¹³C-NMR) gewählt. Daten für die chemische Verschiebung der in Frage kommenden Verbindungen sind der Literatur entnommen (vgL *Die Angewandte Makromolekulare Chemie* **1986**, *141,* 173-183 und darin zit. Lit) bzw. durch Vermessung von Modellsubstanzen gewonnen worden. Das in Anlehnung an das in *Ber*. *d*. *dtsch*. *Chem*. *Ges.* **1927**, *60,* 295 beschriebene Verfahren aus Methylisocyanat unter Katalyse mit ca. 3 % Tri-n-butylphosphin in ca. 70 %iger Ausbeute zugängliche 3,5-Dimethyl-2-methylimino-4,6-diketo-1,3,5-oxadiazin weist folgende NMR-chem. Verschiebungen auf (in ppm): 3,09; 3,08 und 2,84 (¹H-NMR, CH₃) bzw. 148,3; 144,6 und 137,3 (¹³C-NMR, C=O/C=N). Die erfindungsgemäßen Verfahrensprodukte mit Iminooxadiazindionstruktur haben sehr ähnliche ¹³C-NMR chem. Verschiebungen der C=O/C=N Atome und sind zweifelsfrei als solche von anderen Isocyanat-Folgeprodukten zu unterscheiden.

Die dynamischen Viskositäten werden bei 23°C mit dem Viskosimeter VT 550 der Fa. Haake bestimmt. Durch Messungen bei unterschiedlichen Schergeschwindigkeiten ist sichergestellt worden, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Bestimmung der Restmonomergehalte erfolgt gaschromatographisch.

Die Trübung der Trimerisatharze wird mit einem Gerät der Fa. Hach ermittelt. Hierzu werden Streulichtmessungen in 90° zur Richtung eines durch die Harzprobe geleiteten Lichtstrahles der Wellenlänge 400 - 800 nm durchgeführt und in Einheiten bezogen auf Formazin-Standardlösungen, TE(F), angegeben.

Der überwiegende Teil der Reaktionen wird beispielhaft mit HDI als zu trimerisierendem Isocyanat und Katalysatoren auf Basis Tetrabutylphosphoniumhydrogendifluorid unter einer Stickstoffatmosphäre durchgeführt. Das geschieht nur zur Verdeutlichung der Vorteile des erfindungsgemäßen Verfahrens und soll keine Einschränkung der vorliegenden Erfindung auf die beschriebenen Systeme bzw. Reaktionsbedingungen bedeuten.

### Beispiel 1:

### Herstellung von quaternären Phosphoniumpolyfluoriden (Stammlösungen)

In Anlehnung an die in *J. Org*. *Chem.* **1989**, 54, 4827-4829 für die Herstellung ähnlicher Ammoniumverbindungen vorgeschlagene Verfahrensweise.

### a) Bu₄P⁺ F^{-.} n HF in Methanol/iso-Propanol

953,8 g einer 71,4 %igen Bu₄P⁺ Cl⁻-Lösung in iso-Propanol (Cyphos® 443P, Produkt der Fa. Cytec), entsprechend 2,3 mol Bu₄P⁺ Cl⁻ werden in 1 kg techn. Methanol (ca. 0,2 % H₂O) gelöst, mit 150 g (2,58 mol) pulverisiertem Kaliumfluorid versetzt und 24 h bei 20-25°C (Zimmertemperatur) gerührt. Anschließend wird filtriert, der Filterrückstand mit 2 mal 100 g techn. Methanol gewaschen und die vereinigten Filtrate erneut mit 150 g (2,58 mol) pulverisiertem Kaliumfluorid versetzt und 24 h bei 20-25°C (Zimmertemperatur) gerührt. Nach anschließender Filtration und erneutem Waschen mit 2 mal 100 g techn. Methanol wird bei maximal 30°C und einem Druck von ca. 1 mbar am Rotationsverdampfer von überschüssigem Methanol und iso-Propanol weitgehend befreit und erneut filtriert. Die so erhaltene, nahezu farblose Lösung weist folgende Daten auf:
Fluorid (mit ionensensitiver Elektrode bei pH 5,5): 5,0 %
Chlor (gesamt, nach Aufschluß, gravimetrisch): 0,4 %
MeOH (gaschromatografisch, nach Normierung): 16,3 %
*i*-PrOH (gaschromatografisch, nach Normierung): 7,3 %

Zu 100 g dieser Lösung wird unter Rühren und Kühlen (< 20°C) 5,27 g wasserfreies HF portionsweise zugegeben. Nach Abklingen der exothermen Reaktion wird die so gewonnene Tetrabutylphosphoniumhydrogendifluoridlösung (Stammlösung 1, berechneter Fluoridgehalt - F⁻, nicht Gesamt-Fluor- : 4,75 % ) für die unter 3-1 beschriebene Trimerisierung verwendet.

Ein Teil dieser Stammlösung 1 (200g) wird bei maximal 30°C und einem Druck von ca. 1 mbar im Verlauf von ca. 6 h am Rotationsverdampfer bis zur Gewichtskonstanz von Methanol und iso-Propanol noch weitergehend befreit, als das in der Fluoridform unter diesen Bedingungen (Druck, Temperatur) möglich ist. Es resultiert eine farblose Lösung (166 g) die folgende Daten aufweist:
Fluorid (mit ionensensitiver Elektrode bei pH 5,5; unter diesen Bedingungen wird sowohl das ursprünglich als F⁻ vorliegende als auch das als HF zugegebene Fluor als Fluorid, F⁻, detektiert): 10,8 %
HF-Anteil (einfache acidimetrische Titration mit 0,1 n NaOH gegen Phenolphthalein): 5,7 %,
aus diesen beiden Werten errechnet sich ein (formaler) F⁻-Gehalt der Lösung von 5,4 % und ein molares F : HF- Verhältnis von ca. 1:1, d.h. durch das weitere Einengen im Vakuum wird kein HF entfernt
Chlor (gesamt, nach Aufschluß, gravimetrisch): 0,50 %
MeOH (gaschromatografisch, nach Normierung): 3,4 %
*i*-PrOH (gaschromatografisch, nach Normierung): 2,1 %
Viskosität bei 23°C: 280 mPas^{.}s

Diese Mischung ist bei Zimmertemperatur flüssig und erstarrt erst bei der Lagerung im Tiefkühlschrank (- 12°C) zu einer weißen Kristallmasse. Letztere wird selbst bei anschließender Lagerung im Kühlschrank (- 2°C) wieder fast vollständig flüssig (trübe Lösung mit Feststoffpartikeln). Anschließende Lagerung bei Zimmertemperatur (20 - 25°C) liefert erneut die homogene, klare, farblose Lösung mit den o.g. analytischen Daten.

Die so erhaltene, hochkonzentrierte Lösung (im folgenden: Stammlösung 2) wird als solche (Versuch 3-0) sowie in Abmischung mit verschiedenen Alkoholen, mit weiterem HF oder weiterem Phosphoniumfluorid zur HDI-Trimerisierung eingesetzt (vgl. Beispiel 3, Tabelle 1).

### b) Bu₃(C₁₄H₂₉)P⁺ F- in Methanol/iso-Propanol

500 g einer 74,2 %igen Bu₃(C₁₄H₂₉)P⁺ Cl⁻-Lösung in iso-Propanol (Cyphos® 3453P, Produkt der Fa. Cytec), entsprechend 0,85 mol Bu₃(C₁₄H₂₉)P⁺ Cl⁻ werden in 0,5 kg techn. Methanol (ca. 0,2 % H₂O) gelöst, mit 50 g (0,86 mol) pulverisiertem Kaliumfluorid versetzt und 24 h bei 20-25°C (Zimmertemperatur) gerührt. Anschließend wird filtriert, der Filterrückstand mit 2 mal 50 g techn. Methanol gewaschen, die vereinigten Filtrate erneut mit 50 g (0,86 mol) pulverisiertem Kaliumfluorid versetzt und 24 h bei 20-25°C (Zimmertemperatur) gerührt. Nach anschließender Filtration und erneutem Waschen mit 2 mal 50 g techn. Methanol wird bei maximal 30°C und einem Druck von ca. 1 mbar am Rotationsverdampfer von überschüssigem Methanol und iso-Propanol weitgehend befreit und erneut filtriert. Die so erhaltene Lösung weist folgende Daten auf:
Fluorid (mit ionensensitiver Elektrode bei pH 5,5): 3,65 %
Chlor (gesamt, nach Aufschluß, gravimetrisch): 0,145 %
MeOH (gaschromatografisch, nach Normierung): 9,1 %
*i*-PrOH (gaschromatografisch, nach Normierung): 3,8 %

### c) Ph₃(Bu)P⁺ F⁻ in Methanol

20 g (56,3 mmol) Ph₃(Bu)P⁺ Cl⁻ (Fa. Chemconserve) werden in 40 g techn. Methanol (ca. 0,2 % H₂O) gelöst, mit 3,3 g (56,8 mmol) pulverisiertem Kaliumfluorid versetzt und 24 h bei 20-25°C (Zimmertemperatur) gerührt. Anschließend wird filtriert, der Filterrückstand mit 2 mal 5 g techn. Methanol gewaschen und die vereinigten Filtrate erneut mit 3,3 g (56,8 mmol) pulverisiertem Kaliumfluorid versetzt und 24 h bei 20 - 25°C (Zimmertemperatur) gerührt. Nach anschließender Filtration und erneutem Waschen mit 2 mal 5 g techn. Methanol wird bei maximal 30°C und einem Druck von ca. 1 mbar am Rotationsverdampfer bis zur beginnenden Kristallisation von überschüssigem Methanol befreit und erneut filtriert. Dabei ist darauf zu achten, daß nur von Kaliumsalzen, die infolge weiterer Aufkonzentrierung der Lösung ausfallen, abgetrennt wird und kein Phosphoniumsalz im Filterrückstand verbleibt (Löslichkeitsprobe). Die so erhaltene Lösung weist folgende Daten auf:
Fluorid (mit ionensensitiver Elektrode bei pH 5,5): 3,15 %
Chlor (gesamt, nach Aufschluß, gravimetrisch): < 0,2 %
MeOH (gaschromatografisch, nach Normierung): 42,8 %

In Analogie zur Herstellung der Stammlösung 1 aus der zugrundeliegenden Tetrabutylphosphoniumfluoridlösung werden die unter 1b) und 1c) erhaltenen quaternären Phophoniumfluoride durch Zugabe eines Äquivalentes HF in die entsprechenden Hydrogendifluoride überführt und in der unter 3. beschriebenen Weise für HDI-Trimerisierungen eingesetzt (Versuche 3-14, R = -(C₁₃H₂₇)CH₃, und 3-15).

### Beispiel 2:

### Vergleichsbeispiel, nicht erfindungsgemäß

HDI-Trimerisierung unter Verwendung eines quaternären Ammoniumhydrogendifluoridkatalysators **1** (DE-A 19 611 849) **1** wird *gemäß J. Org*. *Chem.* **1989**, *54*, 4827-4829 durch Anionenaustausch aus Aliquat 336 (quaternäres Ammoniumchlorid R₃(Me)N⁺ Cl⁻, R = C₈ - C₁₀ Alkyl, C₈ ist vorherrschend, der Fa. Fluka AG, das Produkt enthielt iso-Propanol) mit KF in MeOH hergestellt und durch anschließende HF-Zugabe, wie in Beispiel 1 beschrieben, in die R₃(Me)N⁺ [HF₂]⁻-Form überführt (F⁻-Gehalt der Lösung: 2,05 %, nicht Gesamtfluor aus HF₂⁻!).

In einem V4A-Reaktor wie in J. *Thermal Anal.* **1983**, *27,* 215-228 beschrieben, werden 320 g (1,9 mol) HDI bei 60°C und einer Tourenzahl des Rührers von 1200 min⁻¹ zunächst durch ca. einstündiges Rühren im Vakuum (0,1 mbar) von gelösten Gasen befreit, mit Stickstoff belüftet und anschließend mit 26 ppm Katalysator 1, bezogen auf die relative Molmasse des Fluoridions (19) und eingesetztes HDI, versetzt (erste Katalysatorzugabe in Abb. 1). Nach 5 min bzw. nochmals nach weiteren 5 min wird die 6 bzw. 3 ppm F⁻ entspechende Menge **1** zugegeben (zweite bzw. dritte Katalysatorzugabe in Abb. 1). Nach insgesamt 15 min wird die Reaktion durch Zugabe von 150 mg Dibutylphosphat abgestoppt und der Reaktionsansatz analysiert. Der Iminooxadiazindionanteil im Trimerengemisch beläuft sich auf 9,5 mol-%. Das nach Dünnschichtdestillation mit einem Labor-Dünnschichtverdampfer, Typ Kurzwegverdampfer, bei 140 °/0,2 mbar erhaltene Trimerisatharz weist den gleichen, niedrigen Iminooxadiazindionanteil auf und ist relativ stark getrübt (10,2 TE(F)).

Versuche zur Reproduktion dieser Ergebnisse führten zu wechselnden, meist ähnlich unbefriedigenden Ergebnissen.

### Beispiel 3:

### Katalyse mit Phosphoniumpolyfluoriden (erfindungsgemäß)

Stammlösung 1 wird, wie in Beispiel 2a beschrieben, im thermokinetischen Reaktor zur HDI-Trimerisierung eingesetzt (Beispiel 3-1 in Tabelle 1; vgl. auch Abb. 2, U_{NCO} ca. 20 %, Abstoppen durch Zugabe der dem F⁻-Verbrauch ensprechenden molaren Menge Dibutylphosphat, F⁻- Bedarf der Reaktion bei 1./2./3. Katalysatorzugabe, vgl. Abb. 2, 40/20/11 ppm F⁻ bezogen auf die Masse an eingesetztem HDI und die relative Masse des Fluoridions F⁻, 19 g/mol, nicht gesamt-Fluor!).

Für die anderen in Tabelle 1 aufgeführten Versuche zur Phosphoniumpolyfluoridkatalysierten HDI-Trimerisierung wird Stammlösung 2, gegebenenfalls mit Alkoholen, HF oder Tetrabutylphosphoniumfluoridlösung versetzt, verwendet. Hierfür werden jeweils 200 g (1,19 mol) HDI in einem 250 ml Vierhalskolben mit Innenthermometer, Rührer, Rückflußkühler, Gaseinleitungsrohr sowie Dosiereinrichtung für die Katalysatorlösung zunächst bei 60°C und einem Druck von ca. 0,1 mbar im Verlauf einer Stunde von im Diisocyanatgemisch gelösten Gasen befreit, anschließend mit Stickstoff belüftet und nachfolgend unter Durchleiten eines schwachen Stickstoffstromes bei 60°C Innentemperatur durch portionsweise Katalysatorzugabe trimerisiert. Der U_{NCO} beträgt jeweils ca. 20 %, abgestoppt wird durch Zugabe der dem F⁻-Verbrauch, nicht gesamt-Fluor, entsprechenden molaren Menge Dibutylphosphat, der F⁻- Bedarf der Reaktion liegt zwischen 10 - 30 ppm F⁻, bezogen auf die Masse an eingesetztem HDI und die relative Masse des Fluoridions (F⁻, 19 g/mol, nicht gesamt-Fluor). Es ist generell, selbst bei Verwendung der hochkonzentrierten Stammlösung 2, keine Feststoffbildung während der Reaktion zu beobachten. Die Iminooxadiazindiongehalte sind Tabelle 1 zu entnehmen.

**Tabelle 1**

| Ergebnisse Phosphoniumpolyfluorid-katalysierter HDI-Trimerisierungen | | | | | |
|---|---|---|---|---|---|
| Versuch Nr. | Alkohol* | Konzentration Bu₄P⁺ F⁻ bzw. R₄P⁺ F⁻[%] (gerundet) | F⁻ : HF im Katalysator (molar) | Harztrübung [TE(F)] | Anteil Iminooxadiazindion im Trimerengemisch [mol-%] |
| 3-0 | MeOH/*i*PrOH | 80 | 1:1 | 0,8 | 51 |
| 3-1 | MeOH/*i*PrOH | 70 | 1:1 | 0,4 | 48 |
| 3-2 | MeOH | 50 | 1:1 | 0,5 | 45 |
| 3-3 | MeOH | 40 | 1:1 | 1,4 | 42 |
| 3-4 | MeOH | 40 | 1:0,5 | 0,8 | 43 |
| 3-5 | *i*PrOH | 50 | 1:1 | 0,5 | 47 |
| 3-6 | *i*PrOH | 40 | 1:1 | 0,4 | 42 |
| 3-7 | *i*PrOH | 30 | 1:1 | 0,6 | 41 |
| 3-8 | *i*PrOH | 20 | 1:1 | 0,9 | 35 |
| 3-9 | *n*BuOH | 50 | 1:1 | 0,5 | 44 |
| 3-10 | *n*BuOH | 30 | 1:0,5 | 0,5 | 38 |
| 3-11 | MeOH/*i*PrOH | 62 | 1:5 | 0,5 | 53 |
| 3-12 | MeOH/*i*PrOH | 50 | 1:10 | 0,4 | 59 |
| 3-13 | MeOH/*i*PrOH | 37 | 1:20 | 0,6 | 64 |
| 3-14 | MeOH/*i*PrOH | ca. 83% Bu₃(R)P⁺ [HF₂]⁻ | 1:1 | 0,6 | 43 |
| 3-15 | MeOH | ca. 57 % Ph₃BuP⁺ [HF₂]⁻ | 1:1 | 0,5 | 44 |

| | | | | | |
|---|---|---|---|---|---|
| * bei den Versuchen 3-2 bis 3-10 wird nur der zur weiteren Verdünnung der Polyfluorid-Stammlösung zugesetzte Alkohol erwähnt | | | | | |

### Beispiel 4:

### HDI/IPDIHDI/IPDI-Mischtrimerisierung (erfindungsgemäß)

Eine Mischung aus 100 g (0,59 mol) HDI und 100 g (0,45 mol) Isophorondiisocyanat (IPDI) wird in einem 250 ml Vierhalskolben mit Innenthermometer, Rührer, Rückflußkühler, Gaseinleitungsrohr sowie Dosiereinrichtung für die Katalysatorlösung zunächst bei Zimmertemperatur und einem Druck von ca. 0,1 mbar im Verlauf einer Stunde von im Diisocyanatgemisch gelösten Gasen befreit und anschließend unter Durchleiten eines schwachen Stickstoffstromes auf 60°C Innentemperatur erhitzt. Anschließend wird bei dieser Temperatur im Verlaufe von ca. 20 Minuten portionsweise insgesamt die 87 ppm F⁻ entsprechende Menge an Stammlösung 1 so zugetropft, daß die Innentemperatur 70°C nicht überschreitet. Es wird bis zu einem NCO-Gehalt der Mischung von 34,0 % trimerisiert, durch Zugabe von 150 mg Di-n-butylphosphat abgestoppt, eine weitere Stunde bei 70°C nachgerührt und anschließend von nicht umgesetzten monomeren Diisocyanaten durch Dünnschichtdestillation in einem Kurzwegverdampfer bei 0,15 mbar und einer Temperatur des Heizmediums von 180°C abgetrennt. Das so erhaltene klare (Trübung = 0,9 TE(F)) und nahezu farblose Harz (66 g entsprechend 33 % Ausbeute) weist in reiner Form eine Viskosität von 23 800 mPa·s, einen NCO-Gehalt von 20,2 % und Restmonomergehalte von 0,03 % an HDI und 0,11 % an IPDI auf. Das molare Verhältnis von Iminooxadiazindionen zu Isocyanuraten beträgt ca. 45:55.

### Beispiel 5:

### Trimerisierung von H₆-XDI (erfindungsgemäß)

100 g (0,51 mol) 1,3-Bis(isocyanatomethyl)cyclohexan (H₆-XDI, Fa. Aldrich) werden zunächst wie in Beispiel 4 beschrieben vorbehandelt und anschließend durch portionsweise Zugabe der Stammlösung 1 (summarisch 42 ppm F⁻) bei 58-60°C innerhalb von 3 Stunden auf einen NCO-Gehalt von 36,4 % trimerisiert. Anschließend wird durch Zugabe von 100 mg Di-n-octylphosphat abgestoppt, eine weitere Stunde bei 60°C nachgerührt und von nicht umgesetztem 1,3-Bis(isocyanatomethyl)cyclohexan durch Dünnschichtdestillation in einem Kurzwegverdampfer bei 0,15 mbar und einer Temperatur des Heizmediums von 150°C abgetrennt. Das so erhaltene, klare und nahezu farblose Harz (34 g entsprechend 34 % Ausbeute) weist einen NCO-Gehalt von 19,7 % auf und ist in reiner Form bei Zimmertemperatur (20-25°C) gerade noch fließfähig. Die Viskosität der 80 %igen Lösung in n-Butylacetat beträgt 1570 mPa·s bei einem NCO-Gehalt von 15,8 %. Der Restmonomergehalt beläuft sich auf 0,03 % an 1,3-Bis(isocyanatomethyl)cyclohexan (H₆-XDI) bei einem Iminooxadiazindionanteil im Trimerengemisch von 52 %.

## Patentansprüche

1. Verfahren zur Herstellung von Trimerisat-Polyisocyanaten mit mindestens 30 mol-% an Iminooxadiazindiongruppen (asymmetrische Trimere) im Trimerengemisch durch katalytisch induzierte Trimerisierung organischer Dioder Polyisocyanate mit einem (mittleren) Molekulargewicht von 140 - 600 g/mol mit unabhängig voneinander aliphatisch, cycloaliphatisch und/oder araliphatisch gebundenen Isocyanatgruppen, **dadurch gekennzeichnet, daß** als Trimerisierungskatalysator quaternäre Phosphoniumfluoride der allgemeinen Formel R₄P⁺ F^{- .} n(HF), wobei R für gleiche oder verschiedene, gegebenenfalls verzweigte, aliphatische, aromatische und/oder araliphatische C₁-C₂₀ Reste steht und gegebenenfalls zwei oder mehrere Substituenten R untereinander und mit dem Phosphoratom auch gesättigte oder ungesättigte Cyclen bilden können und n beliebige Werte zwischen 0,1 und 20 annehmen kann in Abmischung mit Alkohol(gemische)en eines (mittleren) Molekulargewichtsbereiches von 32 - 250 g/mol eingesetzt werden und die Konzentration des quaternären Phosphoniumpolyfluorides 10 Masse-% in der Mischung nicht unterschreitet.

2. Verfahren gemäß Anspruch 1, **dadurch gekennzeichnet, daß** als zu trimerisierende Isocyanatkomponente aliphatische Diisocyanate eines Molekulargewicht(sbereich)es von 140 - 300 g/mol als reine Verbindungen oder in beliebiger Abmischung untereinander eingesetzt werden wobei die Verfahrensprodukte mindestens 35 % an Iminooxadiazindiongruppen (asymmetrische Trimere) im Trimerengemisch enthalten.

3. Verfahren gemäß Anspruch 1, **dadurch gekennzeichnet, daß** als zu trimerisierende Isocyanatkomponente Hexamethylendiisocyanat (HDI), Bis(isocyanatomethyl)cyclohexan (H₆XDI) oder Bis(isocyanatomethyl)norbornan (NBDI) rein oder in beliebiger Mischung untereinander eingesetzt werden.

4. Verfahren gemäß Anspruch 1, **dadurch gekennzeichnet, daß** die Verfahrensprodukte mindestens 35 mol-% an Iminooxadiazindiongruppen (asymmetrische Trimere) im Trimerengemisch enthalten.

## Claims

1. Process for the preparation of trimer polyisocyanates that contain at least 30 mol.% iminooxadiazinedione groups (asymmetric trimers) in the trimer mixture, by the catalytically induced trimerisation of organic di- or poly-isocyanates that have a (an average) molecular weight of from 140 to 600 g/mol. and contain, independently of one another, aliphatically, cycloaliphatically and/or araliphatically bonded isocyanate groups, **characterised in that** there are used as the trimerisation catalyst quaternary phosphonium fluorides of the general formula R₄P⁺ F⁻ n(HF), wherein R represents identical or different, optionally branched, aliphatic, aromatic and/or araliphatic C₁-C₂₀ radicals and, optionally, two or more substituents R may also form, with one another and with the phosphorus atom, saturated or unsaturated rings and n may have any desired value from 0.1 to 20 in admixture with alcohols/alcohol mixtures having a (an average) molecular weight in the range of from 32 to 250 g/mol., and the concentration of the quaternary phosphonium polyfluoride in the mixture is not less than 10 wt.%.

2. Process according to claim 1, **characterised in that** there are used as the isocyanate component to be trimerised aliphatic diisocyanates having a molecular weight (range) of from 140 to 300 g/mol., in the form of pure compounds or in any desired mixture with one another, the products of the process containing at least 35 % iminooxadiazinedione groups (asymmetric trimers) in the trimer mixture.

3. Process according to claim 1, **characterised in that** there is used as the isocyanate component to be trimerised hexamethylene diisocyanate (HDI), bis(iso-cyanatomethyl)cyclohexane (H₆XDI) or bis(isocyanatomethyl)norbornane (NBDI) in pure form or in any desired mixture with one another.

4. Process according to claim 1, **characterised in that** the products of the process contain at least 35 mol.% iminooxadiazinedione groups (asymmetric trimers) in the trimer mixture.

## Revendications

1. Procédé pour la préparation de polyisocyanates trimères comprenant des groupes iminooxadiazinedione (trimères asymétriques) à raison d'au moins 30 moles % dans le mélange trimère, par trimérisation induite par voie catalytique de diisocyanates ou de polyisocyanates organiques possédant un poids moléculaire (moyens) de 140 - 600 g/mole comprenant des groupes isocyanates liés, indépendamment l'un de l'autre à des radicaux aliphatiques, cycloaliphatiques et/ou araliphatiques, **caractérisé en ce qu'**on met en oeuvre, à titre de catalyseur de la trimérisation, des fluorures de phosphonium quaternaires répondant à la formule générale R₄P⁺F^{-.}n(HF) dans laquelle R représente des radicaux aliphatiques, aromatiques et/ou araliphatiques en C₁-C₂₀ identiques ou différents, le cas échéant ramifiés et, le cas échéant, deux substituants R ou plus pouvant former entre eux et avec l'atome de phosphore également des cycles saturés ou insaturés, et n peut prendre n'importe quelle valeur entre 0,1 et 20, en mélange avec des (mélanges d')alcools d'un domaine de poids moléculaire (moyen) de 32 à 250 g/mole, la concentration du polyfluorure de phosphonium quaternaire n'étant pas inférieure à 10 % en masse dans le mélange.

2. Procédé selon la revendication 1, **caractérisé en ce qu'**on met en oeuvre, à titre de composant d'isocyanate à trimériser, des diisocyanates aliphatiques d'un (domaine de) poids moléculaire de 140 à 300 g/mole sous la forme de composés purs ou sous la forme de n'importe quels mélanges de plusieurs d'entre eux, les produits issus du procédé contenant des groupes iminooxadiazinedione (trimères asymétriques) dans le mélange trimère à raison d'au moins 35 %.

3. Procédé selon la revendication 1, **caractérisé en ce qu'**on met en oeuvre, à titre de composant d'isocyanate à trimériser, l'hexaméthylène-diisocyanate (HDI), le bis(isocyanatométhyl)-cyclohexane (H₆XDI) ou le bis(isocyanatométhyl)-norbornane (NBDI) à l'état pur ou sous la forme de n'importe quels mélanges de plusieurs d'entre eux.

4. Procédé selon la revendication 1, **caractérisé en ce que** les produits issus du procédé contiennent des groupes iminooxadiazinedione (trimères asymétriques) dans le mélange trimère à raison d'au moins 35 moles %.
